# EUROPEAN PATENT SPECIFICATION

(11) **EP 4 387 685 B1**
(45) Date of publication and mention of the grant of the patent: **30.04.2025**
(21) Application number: 22764985.2
(22) Date of filing: 18.08.2022
(51) Int. Cl.: A61M 5/142, A61M 5/172, A61M 5/168

(54) **INJECTRODE WITH MULTIPLE ELECTRICAL SENSORS**
INJEKTIONSSTANGE MIT MEHREREN ELEKTRISCHEN SENSOREN
PRODUIT D'INJECTION AVEC DE MULTIPLES CAPTEURS ÉLECTRIQUES

(30) Priority: 18.08.2021 US 202163260376 P
(43) Date of publication of application: 26.06.2024
(73) Proprietor: DIXI Neurolab, Inc., Oxford, MI 48371 (US)
(72) Inventor: GALE, John Thomas, Myrtle Beach, SC 29577 (US)
(74) Representative: DTS Patent- und Rechtsanwälte PartmbB
(86) International application number: PCT/US2022/075108
(87) International publication number: WO 2023/023575

(56) References cited:
- EP-A1- 1 462 141
- WO-A2-2012/075337
- AU-A1- 2013 245 506
- US-A1- 2002 183 738
- US-A1- 2013 035 560

## Description

### Technical Field

This application relates generally to injectrodes.

### Background

Chronic back pain affects millions of people. One approach to treating chronic back pain is through neuromodulation to damage or destroy the spinal nerves that carry the pain impulses to the brain. Another approach is to stimulate these spinal nerves.

A recent focus of treating chronic back pain is the dorsal root ganglia (DRG) 10, which is illustrated schematically in **Fig. 1****.** A detailed view of the DRG 10 is illustrated in **Fig. 2****.** US 2002/183738 A1 treats tissue inside a patient's body and shows how to deliver a tissue-damaging agent to selected areas of the heart. In one example a method of treating cardiac arrhythmia is shown. This method includes positioning a distal tissue-contacting portion of a body in surface contact with a tissue surface of cardiac tissue; detecting the surface contact between the tissue-contacting portion and the tissue surface; and thereafter, injecting a tissue-ablating agent into the cardiac tissue through the tissue-contacting portion of the body.

### Summary

Example embodiments described herein have innovative features, no single one of which is indispensable or solely responsible for their desirable attributes. The following description and drawings set forth certain illustrative implementations of the disclosure in detail, which are indicative of several exemplary ways in which the various principles of the disclosure may be carried out. The illustrative examples, however, are not exhaustive of the many possible embodiments of the disclosure. Without limiting the scope of the claims, some of the advantageous features will now be summarized. Other objects, advantages and novel features of the disclosure will be set forth in the following detailed description of the disclosure when considered in conjunction with the drawings, which are intended to illustrate, not limit, the invention.

The invention is defined in independent device claim 1. Further embodiments are defined in dependent claims 2-8. References to methods below are not part of the claimed invention as such, but are useful for the general understanding of the invention. An aspect of the invention is directed to an apparatus comprising: an elongated body having a tapered distal end and a proximal end that are aligned along an axis; distal and proximal electrical sensors disposed on the elongated body; an injection port on the elongated body between the distal and proximal electrical sensors; a power source electrically coupled to the distal and proximal electrical sensors; a computer having an input electrically coupled to the distal and proximal electrical sensors to receive first and second output signals, respectively, from the distal and proximal electrical sensors; and a non-transitory computer-readable memory operatively coupled to the computer, the non-transitory computer-readable memory storing computer-readable instructions that, when executed by the computer, cause the computer to: analyze an electrical characteristic measured by the distal and proximal electrical sensors; and produce an output control signal when the electrical characteristic measured by the distal and proximal electrical sensors indicates that the injection port is aligned with a target anatomical feature.

In one or more embodiments, the apparatus further comprises a reservoir disposed in the body and in fluid communication with the injection port, the reservoir configured to hold a chemical therapeutic agent, the chemical therapeutic agent configured to damage one or more nerves in the target anatomical feature. In one or more embodiments, the apparatus further comprises an electromechanical valve disposed between the reservoir and the injection port. In one or more embodiments, the electromechanical valve is in electrical communication with the computer, the electromechanical valve having a default closed state in which the reservoir is fluidly decoupled from the injection port and an open state in which the reservoir is fluidly coupled to the injection port, the electromechanical valve configured to transition from the default closed state to the open state in response to the output control signal.

In one or more embodiments, the injection port is aligned with the target anatomical feature when the electrical characteristic measured by the distal and proximal electrical sensors has about the same magnitude. In one or more embodiments, the injection port is aligned with the target anatomical feature when the electrical characteristic measured by the distal and proximal electrical sensors indicates that the distal and proximal electrical sensors are in physical contact with the target anatomical feature.

In one or more embodiments, the injection port is aligned with the target anatomical feature when the magnitude of the electrical characteristic measured by the distal and proximal electrical sensors is higher than a respective baseline magnitude. In one or more embodiments, the first electrical sensor is located closer to the tapered distal end than the second electrical sensor, and the injection port is aligned with the target anatomical feature when the electrical characteristic measured by the first electrical sensor indicates that the first electrical sensor has passed over the target anatomical feature and the electrical characteristic measured by the second electrical sensor indicates that the second electrical sensor is not in physical contact with the target anatomical feature.

In one or more embodiments, the electrical characteristic comprises a resistance or an impedance. In one or more embodiments, the proximal end is flared.

Another aspect of the invention is directed to a method comprising: inserting an apparatus into a mammalian subject, the apparatus comprising: an elongated body having a tapered distal end and a proximal end that are aligned along an axis; first and second electrical sensors disposed on the elongated body; and an injection port on the elongated body between the first and second electrical sensors, wherein: the first and second electrical sensors are electrically coupled to a power source, and the first and second electrical sensors are electrically coupled to an input of a computer to receive first and second output signals, respectively, from the first and second electrical sensors. The method further comprises: monitoring, with the computer, an electrical characteristic measured by the first and second electrical sensors; and producing, with the computer, an output control signal when the electrical characteristic measured by the first and second electrical sensors indicates that the injection port is aligned with a target anatomical feature.

In one or more embodiments, the method further comprises automatically injecting a chemical therapeutic agent, through the injection port, in response to the output control signal. In one or more embodiments, the method further comprises opening an electromechanical valve, in response to the output control signal, to fluidly couple the injection port to a reservoir in the apparatus, the reservoir storing the chemical therapeutic agent. In one or more embodiments, the method further comprises damaging at least one nerve in the target anatomical feature.

In one or more embodiments, the method further comprises: determining, with the computer, when a magnitude of the electrical characteristic measured by the first and second electrical sensors is higher than the magnitude of a respective baseline output signal of the first and second electrical sensors; and producing the output control signal when the magnitude of the electrical characteristic measured by the first and second electrical sensors is higher than the magnitude of the respective baseline output signal of the first and second electrical sensors. In one or more embodiments, the method further comprises producing the output control signal when the magnitude of the electrical characteristic measured by the first and second electrical sensors is about the same and higher than the magnitude of the respective baseline output signal of the first and second electrical sensors.

In one or more embodiments, the method further comprises: (a) determining, with the computer, when a magnitude of the electrical characteristic measured by the first electrical sensor is higher than the magnitude of a baseline output signal of the first electrical sensor and the magnitude of the electrical characteristic measured by the second electrical sensor is about equal to the magnitude of a baseline output signal of the second electrical sensor; (b) after step (a), determining, with the computer, when the magnitude of the electrical characteristic measured by the first and second electrical sensors is about equal to the magnitude of the baseline output signals of the first and second electrical sensors, respectively; and (c) producing the output control signal when the magnitude of the electrical characteristic measured by the first and second electrical sensors is about equal to the magnitude of the baseline output signals of the first and second electrical sensors, respectively, wherein the first electrical sensor is located closer to the tapered distal end than the second electrical sensor.

In one or more embodiments, the method further comprises (d) advancing the apparatus distally in the mammalian subject between steps (a) and (b), wherein the apparatus is in a first position in step (a) and in a second position in step (b). In one or more embodiments, the method further comprises: (e) determining, with the computer, when the magnitude of the electrical characteristic measured by the second electrical sensor is higher than the magnitude of the baseline output signal of the second electrical sensor and the magnitude of the electrical characteristic measured by the first electrical sensor is about equal to the magnitude of the baseline output signal of the first electrical sensor; (f) advancing the apparatus distally in the mammalian subject between steps (b) and (e), wherein the apparatus is in a third position in step (e), the second position between the first position and the third position; and (g) retracting the apparatus proximally in the mammalian subject after step (e) to a fourth position, the fourth position between the first position and the third position; (h) after step (g), repeating step (b), wherein step (c) occurs after step (h).

In one or more embodiments, the electrical characteristic comprises a resistance or an impedance.

### Brief Description of the Drawings

For a fuller understanding of the nature and advantages of the concepts disclosed herein, reference is made to the detailed description of preferred embodiments and the accompanying drawings.

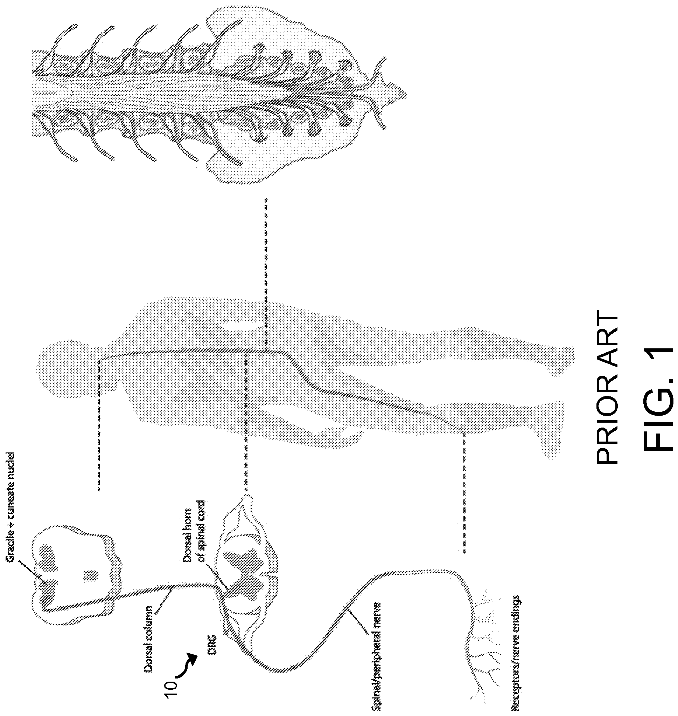

Fig. 1 is a schematic illustration of a dorsal root ganglion (DRG) in a human subject.

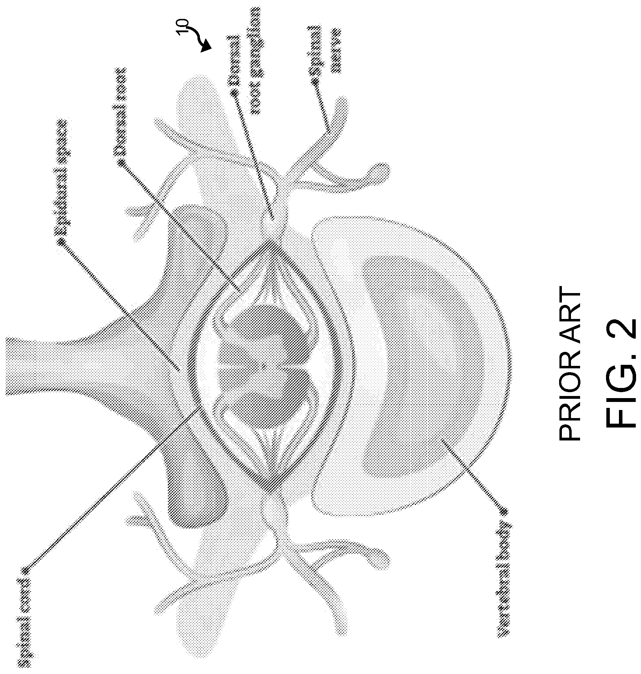

Fig. 2 is a detailed view of the DRG and surrounding anatomy.
Fig. 3 is a simplified view of an injectrode according to an embodiment.
Fig. 4 is a simplified view of an injectrode according to another embodiment.
Fig. 5 illustrates the electrical sensors of the injectrode illustrated in Fig. 3 or 4 in physical contact simultaneously with the DRG according to an embodiment.
Fig. 6 is an example graph of a measured electrical characteristic versus time of the electrical sensors as the injectrode is inserted into the surgical site and into the DRG according to the embodiment illustrated in Fig. 5.
Fig. 7 illustrates another embodiment of the injectrode where only one of the electrical sensors can be in physical contact with the DRG.
Fig. 8 is an example graph of a measured electrical characteristic versus time of the electrical sensors as the injectrode is inserted into the surgical site and into the DRG according to the embodiment illustrated in Fig. 7.
Fig. 9 illustrates another embodiment of the injectrode that includes an electromechanical valve.
Fig. 10 is a flow chart of a method for locating and damaging a neural ganglia according to an embodiment.
Figs. 11A and 11B illustrate an injectrode in first and second positions, respectively, according to an embodiment.
Figs. 12A-12D illustrate an injectrode in first, second, third, and fourth positions, respectively, according to another embodiment.

### Detailed Description

An injectrode includes a proximal electrical sensor, a distal electrical sensor, and an injection port. The injection port is located between the proximal and distal electrical sensors. The injectrode is configured to be inserted into a mammalian subject (e.g., a human), for example to locate and damage a target neural ganglium (e.g., the DRG). The electrical sensors are configured to measure an electrical characteristic (e.g., resistance or impedance) as the injectrode is inserted into the subject. The electrical characteristic measured by an electrical sensor changes when that electrical sensor comes into physical contact with the target neural ganglium. The electrical characteristic measured by the electrical sensors is used to determine when the injection port is located within and/or aligned with the target neural ganglium.

In one embodiment, the injectrode is configured so that both electrical sensors can simultaneously physically contact the target neural ganglium. In this embodiment, the magnitude of the electrical characteristic measured by both electrical sensors increases compared to a baseline value or magnitude when the electrical sensors are in physically contact with the target neural ganglium, which indicates that the injection port is located within and/or aligned with the target neural ganglium.

In another embodiment, the injectrode is configured so that only one electrical sensor can physically contact the target neural ganglium at any one time. In this embodiment, the magnitude of the electrical characteristic measured by the distal electrical sensor increases compared to a baseline value or magnitude when the distal electrical sensor has physically contacted the target neural ganglium while the magnitude of the electrical characteristic measured by the proximal electrical sensor remains about equal to the baseline magnitude. As the injectrode is inserted further distally, the magnitude of the electrical characteristic measured by the distal electrical sensor returns to about the baseline magnitude and the magnitude of the electrical characteristic measured by the proximal electrical sensor remains about equal to the baseline magnitude, which indicates that the target neural ganglium is located between the distal and proximal electrical sensors and that the injection port is located within and/or aligned with the target neural ganglium.

The distal and proximal electrical sensors are electrically coupled to a power source, which can be internal or external to the injectrode. In addition, the distal and proximal electrical sensors or their wires/leads can be electrically coupled to a computer that can be programmed to monitor the electrical characteristic measured by the distal and proximal electrical sensors and produce an output control signal when the injection port is located within and/or aligned with the target neural ganglium.

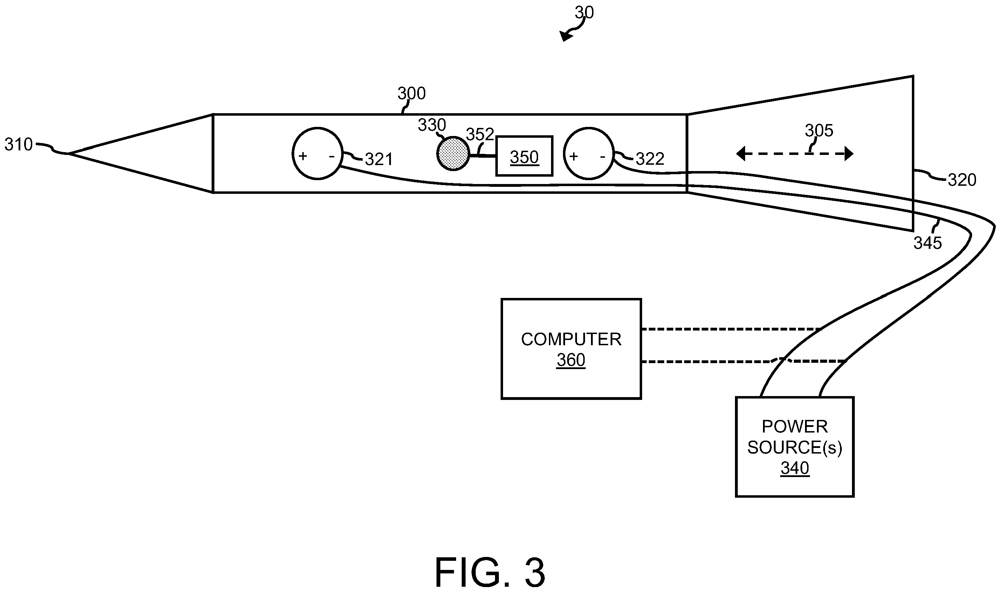

**Fig. 3** is a simplified view of an injectrode 30 according to an embodiment. The injectrode 30 has an elongated body 300, a tapered distal end 310, and a proximal end 320. The tapered distal end 310 is configured to be inserted through a surgical opening in a person's (or another mammalian subject's) back near the DRG. The tapered distal end 310 can terminate in a point, a planar surface, a rounded surface, or another termination. The width of the termination of the tapered distal end 310 is preferably smaller than the width of the body 300. The proximal end 320 can be flared or it can have the same width as the body 300.

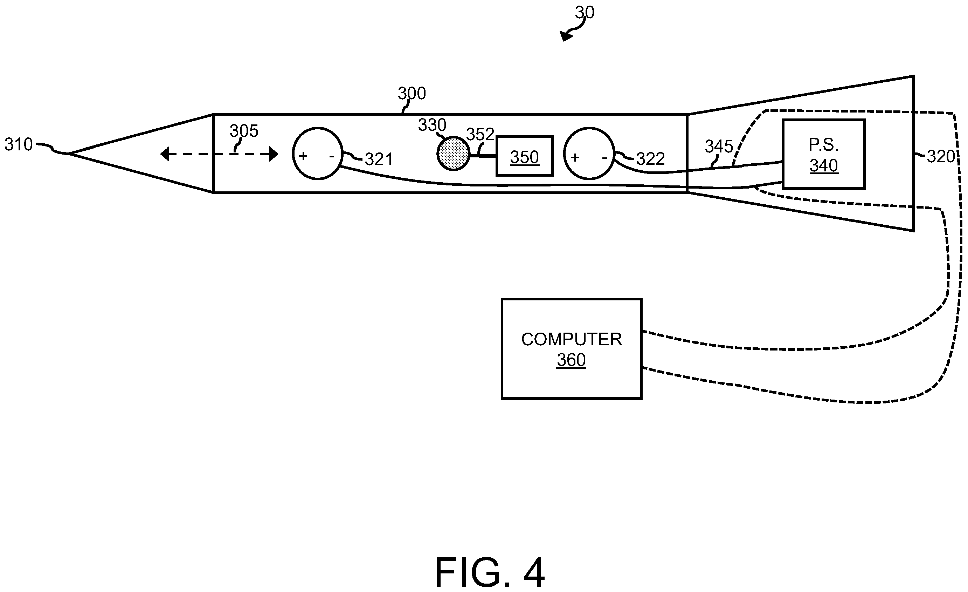

The body 300 includes distal and proximal electrical sensors 321, 322 and an injection port 330. The injection port 330 is located between the electrical sensors 321, 322 along the length of the body 300 (e.g., along a longitudinal axis 305 that extends from the distal end 310 to the proximal end 320). The electrical sensors 321, 322 comprise a conductive material that is preferably biocompatible. For example, the electrical sensors 321, 322 can comprise a biocompatible conductive metal such as platinum or a biocompatible conductive polymer such as a polythiophene (e.g., poly(3,4-ethylenedioxythiophene) (PEDOT)). The electrical sensors 321, 322 are electrically coupled to one or more power sources 340 via respective wires or leads 345 that can extend through and/or be disposed in the proximal end 320 of the injectrode 30. The power source(s) 340 can apply an AC or DC voltage across the positive and negative terminals of each electrical sensor 321, 322. The power source(s) 340 can be an external power source or supply, as illustrated in Fig. 3 or an internal power source or supply, as illustrated in **Fig. 4****.** When the power source(s) 340 is/are an internal power source, the power source(s) 340 can comprise a battery or other internal power supply or source.

The electrical characteristics measured by the electrical sensors 321, 322 can change when the electrical sensors 321, 322 are electrically coupled to different materials. For example, the effective resistance or impedance across the positive and negative terminals of each electrical sensor 321, 322 can vary as the electrical sensors 321, 322 are inserted into the surgical site and placed in physical contact with different anatomical features and substances. The effective resistance or impedance across the positive and negative terminals of the electrical sensor 321, 322 can be relatively low when the electrical sensor 321, 322 is in physical contact with a bodily fluid (e.g., blood) and can be relatively high when the electrical sensor 321, 322 is in physical contact a nerve such as the DRG. Alternatively, the effective resistance or impedance across the positive and negative terminals of the electrical sensor 321, 322 can be relatively high when the electrical sensor 321, 322 is in physical contact with a bodily fluid (e.g., blood) and can be relatively low when the electrical sensor 321, 322 is in physical contact a nerve such as the DRG. Thus, the electrical characteristics measured by the electrical sensors 321, 322 can change when one or both electrical sensors 321, 322 are in physical contact with the DRG, which can indicate when the injection port 330 is positioned in the DRG.

A computer or controller 360 can be electrically coupled to the wires or leads 345 to determine the electrical characteristics (e.g., resistance, impedance, and/or another electrical characteristic) measured by the electrical sensors 321, 322. Additional electrical sensors can be included in other embodiments. Additionally or alternatively, the electrical sensors 321, 322 can be in wireless communication with the computer 360. For example, the electrical sensors 321, 322 and computer 360 can each include a short-range wireless transceiver and antenna that can be compatible and/or support Bluetooth.

When the injection port 330 is positioned in the DRG, a chemical therapeutic agent can be injected into the DRG through injection port 330. The chemical therapeutic agent can be stored in a cavity or reservoir 350 that is fluidly coupled to the injection port 330, such as through a tube 352. The reservoir is disposed in the injectrode 30 such as in the body 300. The chemical therapeutic agent can include a chemical agent, a viral agent, and/or another therapeutic substance such as a genetic-altering (e.g., modulating) agent, a protein-altering (e.g., modulating) agent, and/or a cellular-altering (e.g., modulating) agent. The chemical therapeutic agent can damage or disable some or all nerves in the DRG 10, which can prevent the DRG 10 from transmitting sensory signals to the brain or can limit the magnitude of the sensory signals transmitted to the brain (e.g., to prevent or limit the transmission of pain or other negative sensory signals to the brain).

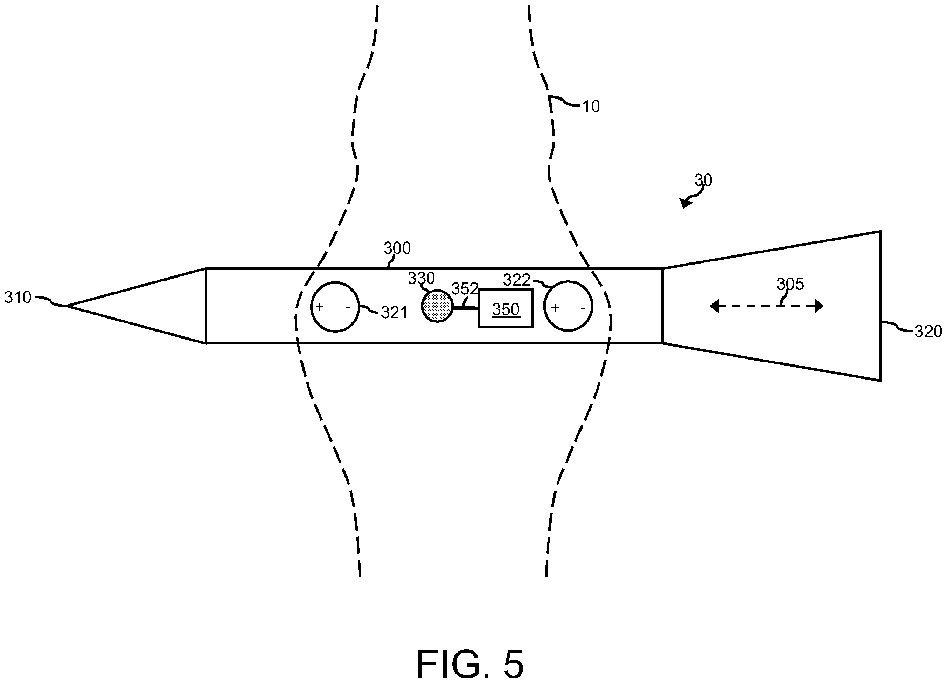

In one embodiment, the electrical sensors 321, 322 are spaced apart along the longitudinal axis 305 such that both electrical sensors 321, 322 can be in physical contact simultaneously with the DRG 10, for example as illustrated in **Fig. 5****.** In this embodiment, the distance along the longitudinal axis 305 between the electrical sensors 321, 322 is less than the thickness of the DRG as measured along the longitudinal axis 305. The wires or leads 345, power source(s) 340, and computer 360 of injectrode 30 are not illustrated in Fig. 5 for clarity purposes.

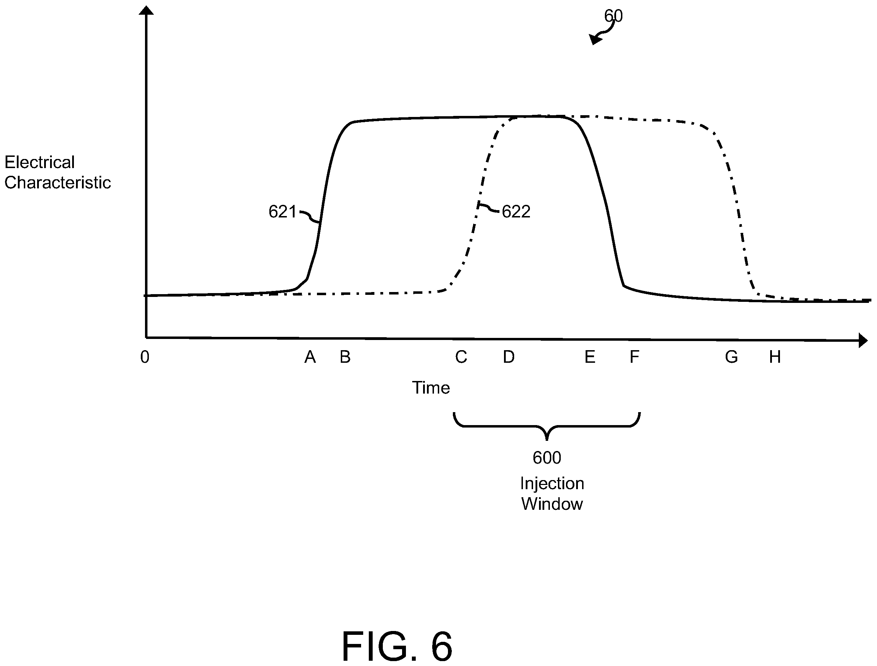

**Fig. 6** is an example graph 60 of a measured electrical characteristic versus time of the electrical sensors 321, 322 according to the embodiment illustrated in Fig. 5 as the injectrode 30 is inserted into the surgical site to the DRG 10. Plot lines 621, 622 are the measured electrical characteristics of the distal and proximal electrical sensors 321, 322, respectively, with respect to time as the injectrode 30 is inserted at a constant rate through a surgical site towards the DRG 10. Since the injectrode 30 is inserted at a constant rate, the times indicated in graph 60 also correspond to relative positions of the injectrode 30. At time 0, plot lines 621, 622 are at a baseline value or magnitude which indicates that neither electrical sensor 321, 322 is in full or partial physical contact with the DRG 10. As can be seen, plot line 621 increases in magnitude at time A, which corresponds to the start of physical contact between a portion of distal electrical sensor 321 and a portion of the DRG 10. Plot line 621 has an increased value or magnitude and is at a plateau at time B, which indicates that the distal electrical sensor 321 is in full physical contact with the DRG 10. Plot line 622 remains at the baseline value or magnitude until time C.

Plot line 622 increases in magnitude at time C (after time A), which corresponds to the start of physical contact between a portion of proximal electrical sensor 322 and a portion of the DRG 10. Plot line 622 has an increased value (e.g., increased magnitude) and is at a plateau at time D, which indicates that the proximal electrical sensor 322 is in full physical contact with the DRG 10. From time D to time E, both plot lines 621, 622 remain at the plateau indicating that both electrical sensors 321, 322 are in full physical contact with the DRG 10 over this time period

Plot line 621 decreases at time E, which indicates that a portion of distal electrical sensor 321 is not in physical contact with the DRG 10. At time F, plot line 621 has returned to the baseline value or magnitude which indicates that the distal electrical sensor 321 is not in partial or full contact with the DRG 10. In other words, the distal electrical sensor 321 has passed through the DRG 10 at time F. Plot line 622 decreases at time G, which indicates that a portion of proximal electrical sensor 322 is not in physical contact with the DRG 10. At time H, plot line 622 has returned to the baseline value or magnitude which indicates that the proximal electrical sensor 322 is not in partial or full contact with the DRG 10. The proximal electrical sensor 322 has passed through the DRG 10 at time H.

It is noted that although graph 60 indicates that the measured electrical characteristic increases in magnitude when an electrical sensor is physical contact with the DRG, in other embodiments, the measured electrical characteristic can decrease in magnitude when the electrical sensor is physical contact with the DRG. The electrical characteristic can comprise electrical resistance, electrical conductivity, electrical impedance, or another electrical characteristic.

The time interval from time C to time F in graph 60 corresponds to the time interval when it can be determined from the plot lines 621, 622 that the injection port 330, in the embodiment illustrated in Fig. 5, is located in the DRG 10. During this time period, the electrical characteristics indicate that one of the electrical sensors is in at least partial physical contact with the DRG and the other electrical sensor is in full physical contact with the DRG. Since the injection port 330 is located between the electrical sensors 321, 322, it can be assured that the injection port 330 is disposed in the DRG during this time interval. This time interval can be referred to as an injection window 600. In summary, the injection window 600 for the embodiment illustrated in Fig. 5 is the time period during which the electrical characteristics measured by the electrical sensors 321, 322, as indicated by plot lines 621, 622, respectively, simultaneously have about the same magnitude (e.g., increased magnitude) that is different than the baseline value or magnitude. As used herein, "about" means plus or minus 10% of the relevant value.

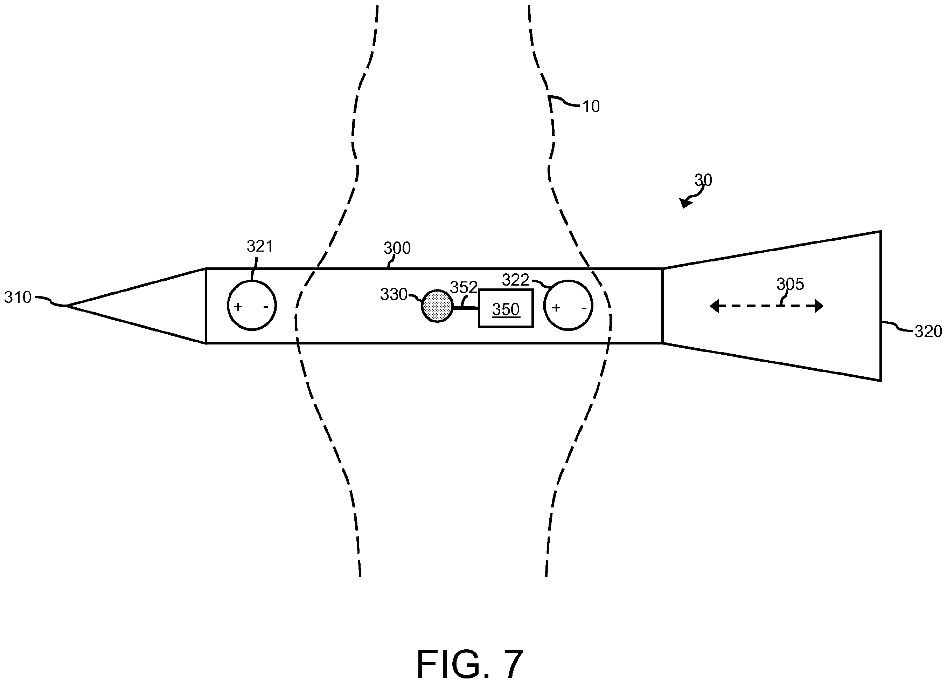

In another embodiment, the electrical sensors 321, 322 are spaced further apart along the longitudinal axis 305 such that only one electrical sensor 321, 322 can be in physical contact with the DRG at any given time, for example as illustrated in **Fig. 7****.** In this embodiment, the distance along the longitudinal axis 305 between the electrical sensors 321, 322 is greater than the thickness of the DRG 10 as measured along the longitudinal axis 305. The wires or leads 345, power source(s) 340, and computer 360 of injectrode 30 are not illustrated in Fig. 7 for clarity purposes.

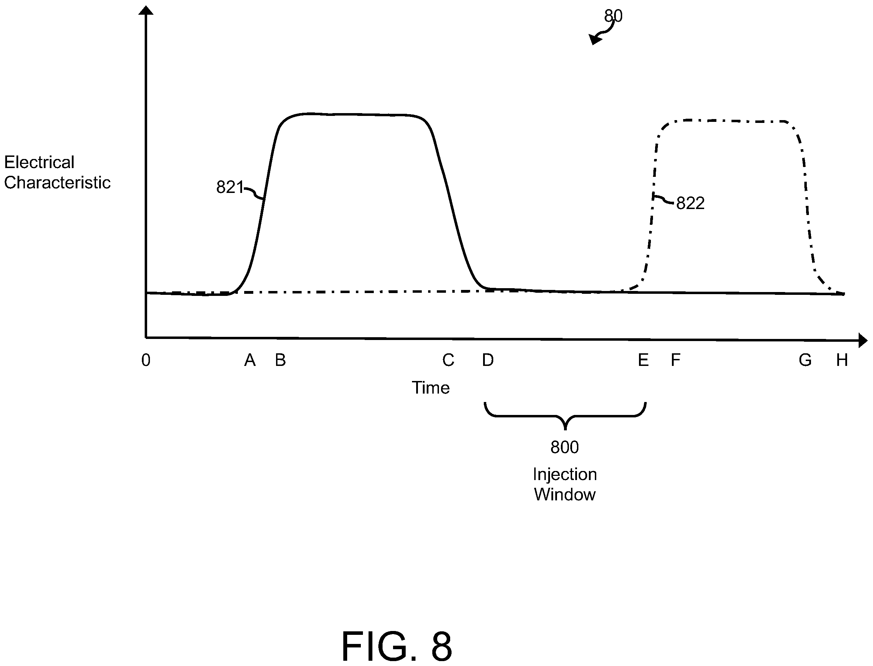

**Fig. 8** is an example graph 80 of a measured electrical characteristic versus time of the electrical sensors 321, 322 according to the embodiment illustrated in Fig. 7 as the injectrode 30 is inserted into the surgical site to the DRG 10. Plot lines 821, 822 are the measured electrical characteristics of the distal and proximal electrical sensors 321, 322, respectively, with respect to time as the injectrode 30 of Fig. 7 is inserted at a constant rate through a surgical site towards the DRG 10. Since the injectrode 30 is inserted at a constant rate, the times indicated in graph 80 also correspond to relative positions of the injectrode 30. At time 0, plot lines 821, 822 are at a baseline value or magnitude which indicates that neither electrical sensor 321, 322 is in full or partial physical contact with the DRG 10. As can be seen, plot line 821 increases in magnitude at time A, which corresponds to the start of physical contact between a portion of distal electrical sensor 321 and a portion of the DRG 10. Plot line 821 has an increased value or magnitude and is at a plateau from time B to time C, which indicates that the distal electrical sensor 321 is in full physical contact with the DRG 10 during this time interval. At time C, the plot line 821 decreases in magnitude, which indicates that a portion of distal electrical sensor 321 is not in physical contact with the DRG 10. At time D, plot line 821 returns to the baseline value or magnitude which indicates that the distal electrical sensor 321 is not in full or partial physical contact with the DRG 10. Thus, at time D the distal electrical sensor 321 has fully passed through the DRG 10.

Plot line 822 remains at the baseline value or magnitude from time 0 until time E when plot line 822 increases in magnitude, which corresponds to the start of physical contact between a portion of proximal electrical sensor 322 and a portion of the DRG 10. The time interval between time D and time E corresponds to the time when the proximal electrical sensor 322 has passed through the DRG 10 but the proximal electrical sensor 322 has not physically contacted the DRG 10. Thus, the distal electrical sensor 321 is on the distal side of the DRG 10 and the proximal electrical sensor 322 is on the proximal side of the DRG 10 during this time interval.

From time F to time G, plot line 822 has an increased value or magnitude and is at a plateau, which indicates that the proximal electrical sensor 322 is in full physical contact with the DRG 10 during this time interval. At time G, the plot line 822 decreases in magnitude, which indicates that a portion of proximal electrical sensor 322 is not in physical contact with the DRG 10. At time H, plot line 822 returns to the baseline value or magnitude which indicates that the proximal electrical sensor 322 is not in full or partial physical contact with the DRG 10. Thus, at time H the proximal electrical sensor 322 has fully passed through the DRG 10.

It is noted that although graph 80 indicates that the measured electrical characteristic increases in magnitude when an electrical sensor is physical contact with the DRG, in other embodiments, the measured electrical characteristic can decrease in magnitude when the electrical sensor is physical contact with the DRG. The electrical characteristic can comprise electrical resistance, electrical conductivity, electrical impedance, or another electrical characteristic.

The time interval from time D to time E in graph 80 corresponds to the time interval when it can be determined from the plot lines 821, 822 that the injection port 330, in the embodiment illustrated in Fig. 7, is located in the DRG 10. During this time period, the electrical characteristics indicate that neither electrical sensor is in at least partial physical contact with the DRG. Since the injection port 330 is located between the electrical sensors 321, 322, it can be assured that the injection port 330 is located in the DRG during this time interval. This time interval can be referred to as an injection window 800. In summary, the injection window 800 for the embodiment illustrated in Fig. 7 is the time period during which the electrical characteristics measured by the electrical sensors 321, 322, as indicated by plot lines 821, 822, respectively, simultaneously have about the same magnitude (e.g., increased magnitude) that is about equal to the baseline value or magnitude.

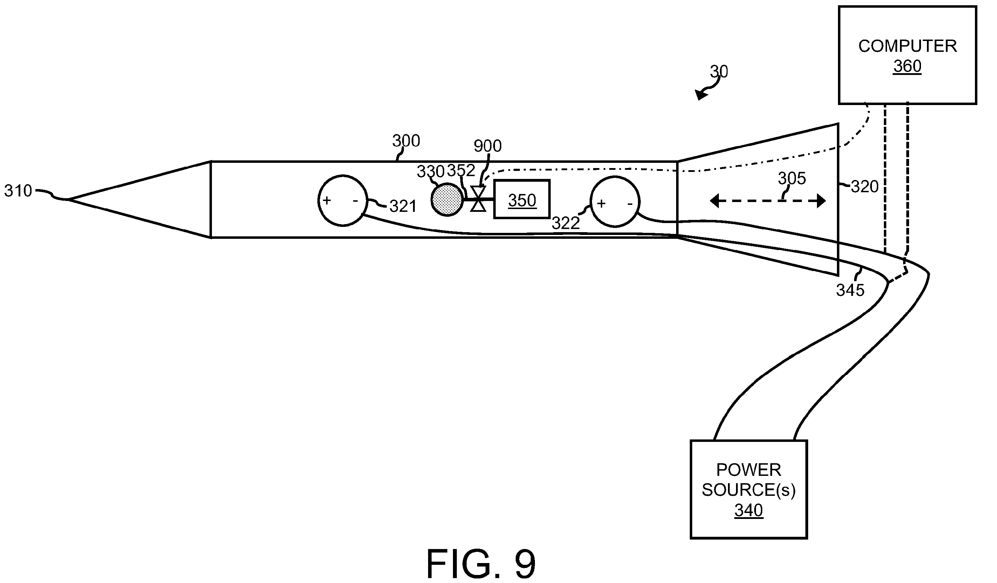

In either embodiment, the computer 360 can be programmed (e.g., in instructions stored in non-volatile memory that is operably coupled to the computer 360 such as operably coupled to a microprocessor of the computer 360) to generate an output control signal to indicate when the injectrode 30 is positioned in the injection window 600, 800 such that the injection port 330 is located in and/or aligned with the DRG. The output control signal can include an audible signal, a visual signal, a haptic signal, and/or other feedback signal to the user. In some embodiments, the injectrode 30 can include an electrical or electromechanical interlock that prevents the injection port from activating until and/or unless the injectrode 30 is positioned in the injection window. For example, an electromechanical valve 900 can be disposed between and fluidly coupled to the reservoir 350 and the injection port 330, as illustrated in **Fig. 9****.** The electromechanical valve 900 can have a default state of closed such that the chemical therapeutic agent in reservoir 350 is not fluidly coupled to the injection port 330. When the computer 360 determines that the injectrode 30 is positioned in the injection window, the computer 360 can send a signal that changes the state of the electromechanical valve 900 from closed to open to fluidly couple the chemical therapeutic agent to injection port 330, allowing the chemical therapeutic agent to be injected into the DRG. The computer 360 can be in wired communication with the electromechanical valve 900 (as illustrated in Fig. 9) and/or in wireless communication with the electromechanical valve 900.

In addition to the above, the electrical sensors 321, 322 can measure or detect a sensory signal in the DRG which can be used to confirm that one or both electrical sensors 321, 322 are positioned in the DRG and not in another anatomical feature. When the injectrode 30 is positioned in an anatomical feature that the operator believes is the DRG, the operator (or another person) can produce a sensory signal by mechanically stimulating (e.g., by touching or stroking) and/or electrically stimulating (e.g., using an electrode) the shin or fingers of the subject, which can cause electrical signals to pass through the sensory nerves in the DRG. If one or both electrical sensors 321, 322 detects these electrical signals, it can be determined that the electrical sensor(s) is/are located in the DRG. If neither electrical sensor detects the electrical signals, then it can be determined that the electrical sensors are not located in the DRG.

In some situations, more than one DRG can be connected to the anatomical feature where the subject feels pain (or other undesired sensory signal). In these situations, damaging/disabling one DRG may not remove (or fully remove) the subject's pain symptoms. To make sure that all DRGs associated with the subject's pain symptoms are treated, the injectrode 30 can be used to inject a temporary numbing agent (e.g., via injection port 330 or another port), such as lidocaine, into a DRG after the operator confirms that the DRG contributes to carrying the undesirable pain signals to the brain. The operator can confirm that the DRG contributes to carrying the undesirable pain signals by mechanically and/or electrically stimulating the location on the subject's body where pain is felt and then detecting/measuring any corresponding electrical signals in the patient's spinal cord. After the DRG is temporarily numbed with lidocaine, the operator can restimulate the same location on the subject's body to determine if corresponding electrical signals are still detected in the patient's spinal cord. If electrical signals in the patient's spinal cord are still detected in response to the second stimulation, the operator can confirm that a second DRG may need to be treated. When the operator locates another DRG, the operator can confirm whether the second DRG contributes to carrying the undesirable pain signals using the same procedure as in the first DRG. If the operator confirms that the second DRG contributes to carrying the undesirable pain signals, the second DRG can be temporarily numbed with lidocaine. Then the operator can restimulate the same location on the subject's body to determine if any corresponding electrical signals are detected in the patient's spinal cord. If such electrical signals are detected, the process repeats (additional DRGs are located and temporarily numbed) until the stimulation does not induce electrical signals in the patient's spinal cord. When no electrical signals are induced in the spinal cord in response to simulating the location on the subject's body, the operator can confirm that he/she has located all DRGs that contribute to carrying the undesirable pain signals to the brain. These DRG(s) can then be treated with a chemical therapeutic agent, as described above.

In addition to DRGs, the device and method can be used in other neural ganglia (facial ganglia, stellate ganglia, nodose ganglia, etc.), in brain targets (e.g., Parkinson's-knowing physiologically that you are in a specific location of the target, epilepsy, etc.), and/or in other tissues of the body based on electrical properties (e.g., in a specific part of liver, in a vein and out or through the vein, subcutaneous -v- intramuscular, etc.).

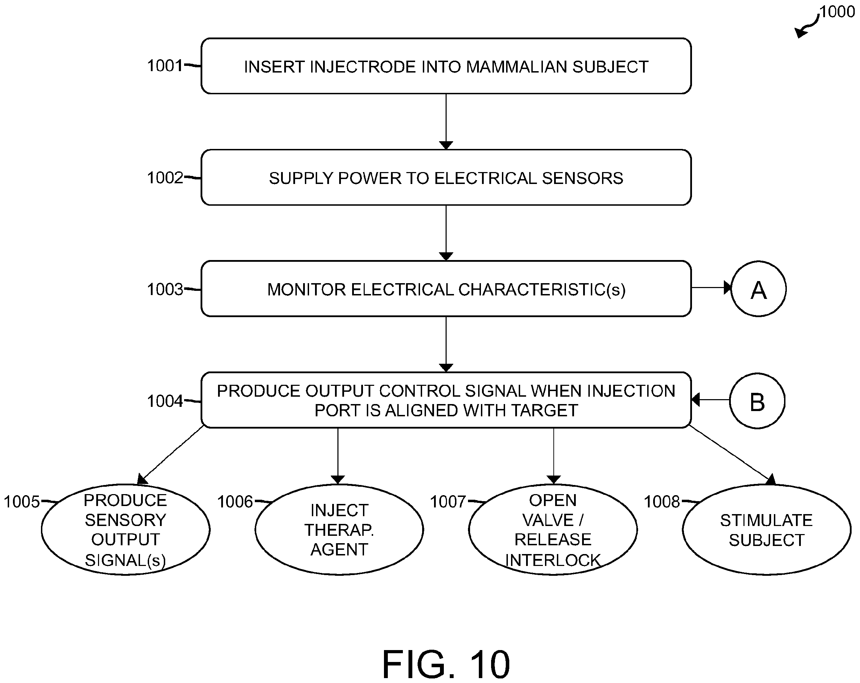

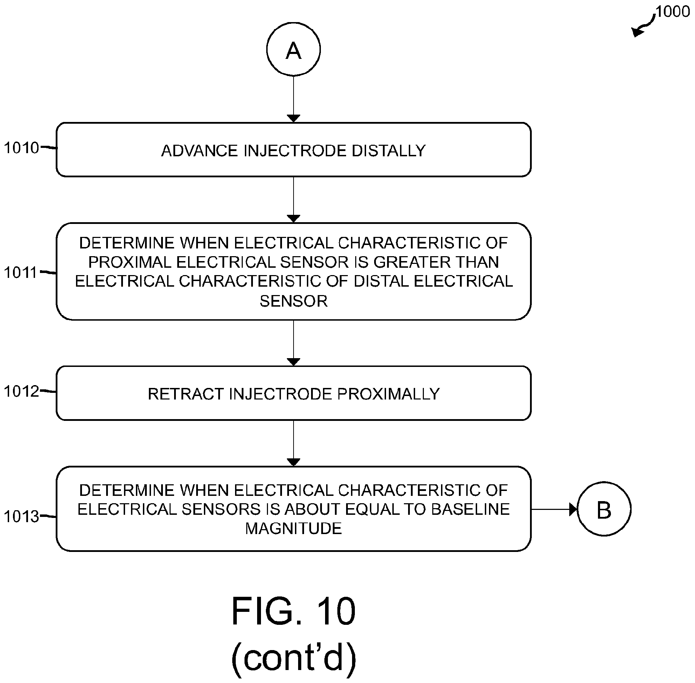

**Fig. 10** is a flow chart of a method 1000 for locating and damaging a neural ganglium according to an embodiment. In step 1001, the injectrode 30 is inserted into a mammalian subject in the region of the target anatomical feature. A different injectrode or apparatus can be inserted into the mammalian subject in another embodiment. The injectrode can be inserted into a surgical site or into a natural body orifice.

In step 1002, power is supplied to the electrical sensors 321, 322 on the injectrode. The power can be supplied from an internal or external power source, such as power source(s) 340. The power can be AC power or DC power, which can produce an AC voltage or a DC voltage, respectively, across the positive and negative electrical terminals of each electrical sensor 321, 322.

In step 1003, one or more electrical characteristics measured by the electrical sensors 321, 322 is/are monitored with a computer 360 that is electrically coupled to the electrical sensors 321, 322. For example, the computer 360 can be electrically coupled to the wires or leads 345 that electrically couple the power source(s) 340 to the electrical sensors 321, 322. Additionally or alternatively, the computer 360 can be in wireless communication with the electrical sensors 321, 322.

In step 1004, the computer 360 produces an output control signal when the computer determines that the electrical characteristic(s) measured by the electrical sensors 321, 322 correspond to an injection window (e.g., injection window 600 or injection window 800) in which the injection port 330 is aligned with and/or located in the neural ganglia (e.g., DRG). The output control signal can include or produce one or more sensory output signals in step 1005 to alert a user or operator. For example, the output control signal can include an output audio signal, an output video/graphical signal, and/or an output haptic signal. Additionally or alternatively, the output control signal can cause the injectrode 30 to automatically inject a chemical therapeutic agent through the injection port 330 in step 1006, which can damage one or more nerves in the target neural ganglium. Additionally or alternatively, the output control signal can cause an electromechanical valve (e.g., electromechanical valve 900) to open in step 1007 to fluidly couple the injection port 330 to the reservoir 350, which can allow the chemical therapeutic agent to be injected through the injection port 330. Additionally or alternatively, the output control signal can cause an interlock to be released in step 1007 to allow the chemical therapeutic agent to be injected through the injection port 330.

In some embodiments, the operator (or another person) can produce a sensory signal by mechanically stimulating (e.g., by touching or stroking) and/or electrically stimulating (e.g., using an electrode) the shin or fingers of the subject in step 1008, which can cause electrical signals to pass through the sensory nerves in the DRG. If one or both electrical sensors 321, 322 detects these electrical signals, it can be determined that the electrical sensor(s) is/are located in the DRG. If neither electrical sensor detects the electrical signals, then it can be determined that the electrical sensors are not located in the DRG.

Steps 1001-1003 can occur simultaneously such that the injectrode is inserted and being positioned while power is supplied to the electrical sensors and the measured electrical characteristic(s) are being monitored.

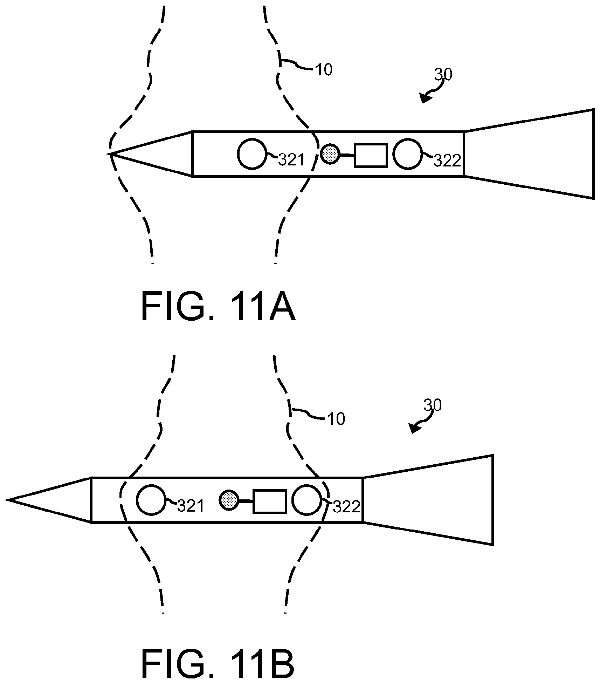

During steps 1001-1003, the injectrode of Fig. 5 is inserted to a first position where the distal electrical sensor 321 is in physical contact with the target neural ganglium (e.g., DRG 10) and the proximal electrical sensor 322 is not in physical contact with the target neural ganglium, as illustrated in **Fig. 11A****.** At the first position, the magnitude of the electrical characteristic measured by the distal electrical sensor 321 is higher than the magnitude of the electrical characteristic measured by the distal electrical sensor 322, such as time B to time C in Fig. 6. The injectrode is then inserted further to a second position that is distal to the first position, as illustrated in **Fig. 11B****.** At the second position, the target neural ganglium (e.g., DRG 10) is between the electrical sensors 321, 322 such that the electrical sensors 321, 322 are not in physical contact with the target neural ganglium. At the second position, the magnitude of the electrical characteristic measured by the electrical sensors 321, 322 is low and about equal to the baseline value or magnitude, such as the injection window 800 in Fig. 8.

When the injectrode has the configuration of Fig. 7, additional positioning may be desired after the output control signal is produced in step 1004 to confirm that the injectrode is positioned correctly with respect to the target neural ganglium. For example, referring to Fig. 8 it can be seen that the magnitude of the electrical characteristic during the injection window 800 is about the same as the baseline value or magnitude of the electrical characteristic from time 0 to time A. Therefore it may be desirable to confirm that a low-magnitude electrical characteristic signal measured by both electrical sensors corresponds to the injection window 800.

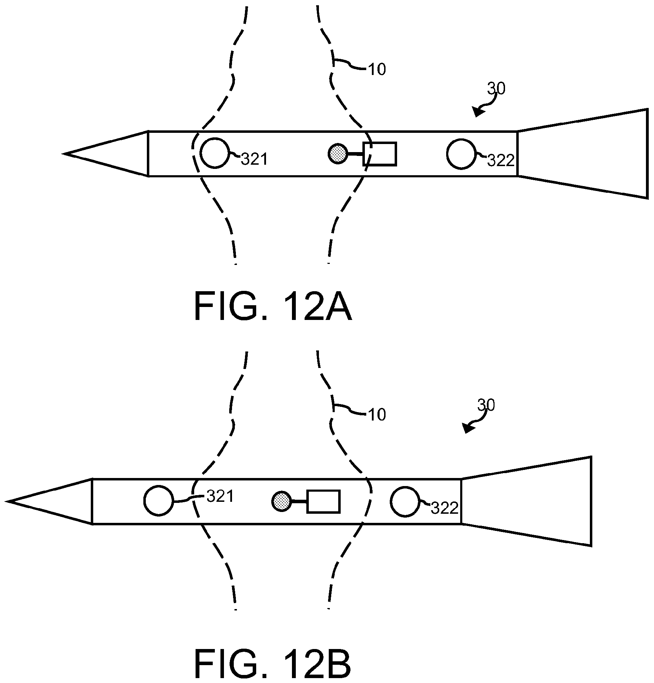

During steps 1001-1003, the injectrode of Fig. 7 is inserted to a first position where the distal electrical sensor 321 is in physical contact with the target neural ganglium and the proximal electrical sensor 322 is not in physical contact with the target neural ganglium (e.g., DRG 10), as illustrated in **Fig. 12A****.** At the first position, the magnitude of the electrical characteristic measured by the distal electrical sensor 321 is higher than the magnitude of the electrical characteristic measured by the proximal electrical sensor 322, such as time B to time C in Fig. 8. The injectrode is then inserted further to a second position that is distal to the first position, as illustrated in **Fig. 12B****.** At the second position, the target neural ganglium (e.g., DRG 10) is between the electrical sensors 321, 322 such that the electrical sensors 321, 322 are not in physical contact with the target neural ganglium. At the second position, the magnitude of the electrical characteristic measured by the electrical sensors 321, 322 is low and about equal to the baseline value or magnitude, such as the injection window 800 in Fig. 8.

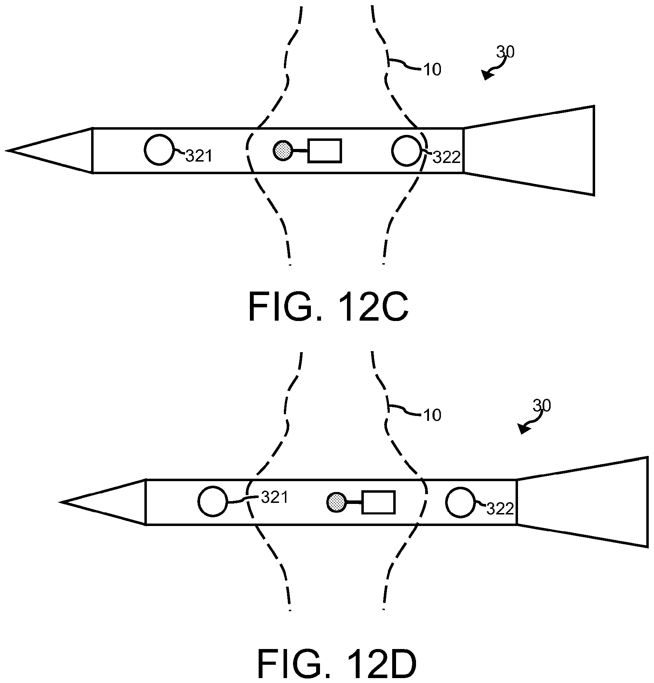

In optional step 1010 (via placeholder A), the injectrode is advanced distally from the second position to a third position where the proximal electrical sensor 322 is in physical contact with the target neural ganglium (e.g., DRG 10) and the distal electrical sensor 321 is not in physical contact with the target neural ganglium, as illustrated in **Fig. 12C****.** At the third position, the magnitude of the electrical characteristic measured by the proximal electrical sensor 322 is higher than the magnitude of the electrical characteristic measured by the distal electrical sensor 321, such as time F to time G in Fig. 8. In optional step 1011, the computer 360 determines when the magnitude of the electrical characteristic measured by the proximal electrical sensor 322 is higher than the magnitude of the electrical characteristic measured by the distal electrical sensor 321. In optional step 1012, the injectrode is retracted proximally from the third position to a fourth position where the target neural ganglium (e.g., DRG 10) is between the electrical sensors 321, 322 such that the electrical sensors 321, 322 are not in physical contact with the target neural ganglium, as illustrated in **Fig. 12D****.** The fourth position is between the first and third positions and can be the same as the second position. At the fourth position, the magnitude of the electrical characteristic measured by the electrical sensors 321, 322 is low and about equal to the baseline value or magnitude, such as the injection window 800 in Fig. 8. In optional step 1013, the computer 360 determines when the magnitude of the electrical characteristic measured by the electrical sensors 321, 322 is about the same and about equal to the baseline value or magnitude. After optional step 1013, the method 1000 returns to step 1004 (via placeholder B) to produce an output control signal.

This disclosure should not be considered limited to the particular embodiments described above. Various modifications, equivalent processes, as well as numerous structures to which the present invention may be applicable, will be readily apparent to those skilled in the art to which the invention is directed upon review of this disclosure. The above-described embodiments may be implemented in numerous ways. One or more aspects and embodiments involving the performance of processes or methods may utilize program instructions executable by a device (e.g., a computer, a processor, or other device) to perform, or control performance of, the processes or methods.

In this respect, various inventive concepts may be embodied as a non- transitory computer readable storage medium (or multiple non-transitory computer readable storage media) (e.g., a computer memory of any suitable type including transitory or non-transitory digital storage units, circuit configurations in field programmable gate arrays (FPGAs) or other semiconductor devices, or other tangible computer storage medium) encoded with one or more programs that, when executed on one or more computers or other processors, perform methods that implement one or more of the various embodiments described above. When implemented in software (e.g., as an app), the software code may be executed on any suitable processor or collection of processors, whether provided in a single computer or distributed among multiple computers.

Further, it should be appreciated that a computer may be embodied in any of a number of forms, such as a rack-mounted computer, a desktop computer, a laptop computer, or a tablet computer, as non-limiting examples. Additionally, a computer may be embedded in a device not generally regarded as a computer but with suitable processing capabilities, including a personal digital assistant (PDA), a smartphone or any other suitable portable or fixed electronic device.

Also, a computer may have one or more communication devices, which may be used to interconnect the computer to one or more other devices and/or systems, such as, for example, one or more networks in any suitable form, including a local area network or a wide area network, such as an enterprise network, an intelligent network, or the Internet. Such networks may be based on any suitable technology and may operate according to any suitable protocol and may include wireless networks or wired networks.

Also, a computer may have one or more input devices and/or one or more output devices. These devices can be used, among other things, to present a user interface. Examples of output devices that may be used to provide a user interface include printers or display screens for visual presentation of output and speakers or other sound generating devices for audible presentation of output. Examples of input devices that may be used for a user interface include keyboards, and pointing devices, such as mice, touch pads, and digitizing tablets. As another example, a computer may receive input information through speech recognition or in other audible formats.

The non-transitory computer readable medium or media may be transportable, such that the program or programs stored thereon may be loaded onto one or more different computers or other processors to implement various one or more of the aspects described above. In some embodiments, computer readable media may be non- transitory media.

The terms "program," "app," and "software" are used herein in a generic sense to refer to any type of computer code or set of computer-executable instructions that may be employed to program a computer or other processor to implement various aspects as described above. Additionally, it should be appreciated that, according to one aspect, one or more computer programs that when executed perform methods of the present application need not reside on a single computer or processor, but may be distributed in a modular fashion among a number of different computers or processors to implement various aspects of the present application.

Computer-executable instructions may be in many forms, such as program modules, executed by one or more computers or other devices. Generally, program modules include routines, programs, objects, components, data structures, etc. that performs particular tasks or implement particular abstract data types. The functionality of the program modules may be combined or distributed as desired in various embodiments.

Also, data structures may be stored in computer-readable media in any suitable form. For simplicity of illustration, data structures may be shown to have fields that are related through location in the data structure. Such relationships may likewise be achieved by assigning storage for the fields with locations in a computer-readable medium that convey relationship between the fields. However, any suitable mechanism may be used to establish a relationship between information in fields of a data structure, including through the use of pointers, tags or other mechanisms that establish relationship between data elements.

Thus, the present disclosure includes new and novel improvements to existing methods and technologies, which were not previously known nor implemented to achieve the useful results described above. Users of the present method and system will reap tangible benefits from the functions now made possible on account of the specific modifications described herein causing the effects in the system and its outputs to its users. It is expected that significantly improved operations can be achieved upon implementation of the invention.

## Claims

1. An apparatus comprising:
an elongated body having a tapered distal end and a proximal end that are aligned along an axis;
distal and proximal electrical sensors (321; 322) disposed along a length of the elongated body and between the tapered distal end and the proximal end;
an injection port (330) on the elongated body between the distal and proximal electrical sensors (321; 322);
a power source (340) electrically coupled to the distal and proximal electrical sensors (321; 322);
a computer having an input electrically coupled to the distal and proximal electrical sensors (321; 322) to receive first and second output signals, respectively, from the distal and proximal electrical sensors (321; 322); and
a non-transitory computer-readable memory operatively coupled to the computer, the non-transitory computer-readable memory storing computer-readable instructions that, when executed by the computer, cause the computer to:
analyze an electrical characteristic measured by the distal and proximal electrical sensors (321; 322); and
produce an output control signal when the electrical characteristic measured by the distal and proximal electrical sensors (321; 322) indicates that the injection port (330) is aligned with a target anatomical feature.

2. The apparatus of claim 1, further comprising a reservoir disposed in the body and in fluid communication with the injection port (330), the reservoir configured to hold a chemical therapeutic agent, the chemical therapeutic agent configured to damage one or more nerves in the target anatomical feature.

3. The apparatus of claim 2, further comprising an electromechanical valve disposed between the reservoir and the injection port (330).

4. The apparatus of claim 3, wherein the electromechanical valve is in electrical communication with the computer, the electromechanical valve having a default closed state in which the reservoir is fluidly decoupled from the injection port (330) and an open state in which the reservoir is fluidly coupled to the injection port (330), the electromechanical valve configured to transition from the default closed state to the open state in response to the output control signal.

5. The apparatus of claim 1, wherein the injection port (330) is aligned with the target anatomical feature when the electrical characteristic measured by the distal and proximal electrical sensors (321; 322) has about the same magnitude.

6. The apparatus of claim 5, wherein the injection port (330) is aligned with the target anatomical feature when the electrical characteristic measured by the distal and proximal electrical sensors (321; 322) indicates that the distal and proximal electrical sensors (321; 322) are in physical contact with the target anatomical feature.

7. The apparatus of claim 6, wherein the injection port (330) is aligned with the target anatomical feature when the magnitude of the electrical characteristic measured by the distal and proximal sensors (321; 322) is higher than a respective baseline magnitude.

8. The apparatus of claim 5, wherein:
the distal electrical sensor is located closer to the tapered distal end than the proximal electrical sensor, and
the injection port (330) is aligned with the target anatomical feature when the electrical characteristic measured by the distal electrical sensor indicates that the distal electrical sensor has passed over the target anatomical feature and the electrical characteristic measured by the proximal electrical sensor indicates that the proximal electrical sensor is not in physical contact with the target anatomical feature.

9. The apparatus of claim 1, wherein the electrical characteristic comprises a resistance or an impedance.

10. The apparatus of claim 1, wherein the proximal end is flared.

## Patentansprüche

1. Vorrichtung, aufweisend:
einen länglichen Körper mit einem sich verjüngenden distalen Ende und einem proximalen Ende, die entlang einer Achse ausgerichtet sind;
einen distalen und einen proximalen elektrischen Sensor (321; 322), die entlang einer Länge des länglichen Körpers angeordnet sind und sich zwischen dem sich verjüngenden distalen Ende und dem proximalen Ende erstrecken;
eine Injektionsöffnung (330) an dem länglichen Körper zwischen dem distalen und proximalen elektrischen Sensor (321; 322);
eine Stromquelle (340), die an den distalen und proximalen elektrischen Sensor (321; 322) angeschlossen ist;
einen Computer mit einem Eingang, der an den distalen und proximalen elektrischen Sensor (321; 322) angeschlossen ist, um von dem distalen und proximalen elektrischen Sensor (321; 322) ein erstes bzw. zweites Ausgangssignal zu empfangen; und
einen nichtflüchtigen computerlesbaren Speicher, der funktionsmäßig an den Computer angeschlossen ist, wobei in dem nichtflüchtigen computerlesbaren Speicher computerlesbare Anweisungen gespeichert sind, die bei Ausführung durch den Computer diesen zu Folgendem veranlassen:
Analysieren einer elektrischen Eigenschaft, der von dem distalen und proximalen elektrischen Sensor (321; 322) gemessen wird; und
Erzeugen eines Ausgangssteuersignals, wenn die von dem distalen und proximalen elektrischen Sensor (321; 322) gemessene elektrische Eigenschaft angibt, dass die Injektionsöffnung (330) mit einem anatomischen Zielmerkmal ausgerichtet ist.

2. Vorrichtung nach Anspruch 1, darüber hinaus ein Reservoir aufweisend, das in dem Körper angeordnet ist und mit der Injektionsöffnung (330) in Fluidverbindung steht, wobei das Reservoir dafür ausgelegt ist, ein chemisches therapeutisches Mittel zu enthalten, wobei das chemische therapeutische Mittel dafür ausgelegt ist, einen oder mehrere Nerven in dem anatomischen Zielmerkmal zu zerstören.

3. Vorrichtung nach Anspruch 2, darüber hinaus ein elektromechanisches Ventil aufweisend, das zwischen dem Reservoir und der Injektionsöffnung (330) angeordnet ist.

4. Vorrichtung nach Anspruch 3, wobei das elektromechanische Ventil mit dem Computer in elektrischer Verbindung steht, wobei das elektromechanische Ventil einen geschlossenen Standardzustand hat, in dem das Reservoir von der Injektionsöffnung (330) fluidisch entkoppelt ist, und einen offenen Zustand, in dem das Reservoir mit der Injektionsöffnung (330) fluidisch verbunden ist, wobei das elektromechanische Ventil dafür ausgelegt ist, im Ansprechen auf das Ausgangssteuersignal von dem geschlossenen Standardzustand in den offenen Zustand überzugehen.

5. Vorrichtung nach Anspruch 1, wobei die Injektionsöffnung (330) mit dem anatomischen Zielmerkmal ausgerichtet ist, wenn die von dem distalen und proximalen elektrischen Sensor (321; 322) gemessene elektrische Eigenschaft in etwa dieselbe Magnitude hat.

6. Vorrichtung nach Anspruch 5, wobei die Injektionsöffnung (330) mit dem anatomischen Zielmerkmal ausgerichtet ist, wenn die von dem distalen und proximalen elektrischen Sensor (321; 322) gemessene elektrische Eigenschaft anzeigt, dass der distale und proximale elektrische Sensor (321; 322) mit dem anatomischen Zielmerkmal in physischem Kontakt sind.

7. Vorrichtung nach Anspruch 6, wobei die Injektionsöffnung (330) mit dem anatomischen Zielmerkmal ausgerichtet ist, wenn die von dem distalen und proximalen Sensor (321; 322) gemessene Magnitude der elektrischen Eigenschaft höher ist als eine jeweilige Basislinien-Magnitude.

8. Vorrichtung nach Anspruch 5, wobei:
sich der distale elektrische Sensor näher an dem sich verjüngenden distalen Ende befindet als der proximale elektrische Sensor, und
die Injektionsöffnung (330) mit dem anatomischen Zielmerkmal ausgerichtet ist, wenn die von dem distalen elektrischen Sensor gemessene elektrische Eigenschaft anzeigt, dass der distale elektrische Sensor über das anatomische Zielmerkmal gefahren ist, und die von dem proximalen elektrischen Sensor gemessene elektrische Eigenschaft anzeigt, dass der proximale elektrische Sensor mit dem anatomischen Zielmerkmal nicht in physischem Kontakt ist.

9. Vorrichtung nach Anspruch 1, wobei die elektrische Eigenschaft einen Widerstand oder eine Impedanz umfasst.

10. Vorrichtung nach Anspruch 1, wobei das proximale Ende aufgeweitet ist.

## Revendications

1. Appareil comprenant :
un corps allongé ayant une extrémité distale effilée et une extrémité proximale qui sont alignées le long d'un axe ;
des capteurs électriques proximaux et distaux (321 ; 322) disposés le long d'une longueur du corps allongé et entre l'extrémité distale effilée et l'extrémité proximale ;
un orifice d'injection (330) prévu sur le corps allongé entre les capteurs électriques proximaux et distaux (321 ; 322) ;
une source d'alimentation en courant (340) couplée électriquement aux capteurs électriques proximaux et distaux (321 ; 322) ;
un ordinateur ayant une entrée couplée électriquement aux capteurs électriques proximaux et distaux (321 ; 322) pour recevoir respectivement les premier et second signaux de sortie, à partir des capteurs électriques proximaux et distaux (321 ; 322) ; et
une mémoire lisible par ordinateur non volatile couplée en fonctionnement à l'ordinateur, la mémoire lisible par ordinateur non volatile mémorisant les instructions lisibles par ordinateur qui, lorsqu'elles sont exécutées par l'ordinateur, amènent l'ordinateur à :
analyser une caractéristique électrique mesurée par les capteurs électriques proximaux et distaux (321 ; 322) ; et
produire un signal de commande de sortie lorsque la caractéristique électrique mesurée par les capteurs électriques proximaux et distaux (321 ; 322) indique que l'orifice d'injection (330) est aligné avec une caractéristique anatomique cible.

2. Appareil selon la revendication 1, comprenant en outre un réservoir disposé dans le corps et en communication fluide avec l'orifice d'injection (330), le réservoir étant configuré pour contenir un agent de traitement chimique, l'agent de traitement chimique étant configuré pour endommager un ou plusieurs nerfs dans la caractéristique anatomique cible.

3. Appareil selon la revendication 2, comprenant en outre une vanne électromécanique disposée entre le réservoir et l'orifice d'injection (330).

4. Appareil selon la revendication 3, dans lequel la vanne électromécanique est en communication électrique avec l'ordinateur, la vanne électromécanique ayant un état fermé par défaut, dans lequel le réservoir est découplé de façon fluide de l'orifice d'injection (330) et un état ouvert dans lequel le réservoir est couplé de façon fluide à l'orifice d'injection (330), la vanne électromécanique étant configurée pour réaliser la transition de l'état fermé par défaut à l'état ouvert en réponse au signal de commande de sortie.

5. Appareil selon la revendication 1, dans lequel l'orifice d'injection (330) est aligné avec la caractéristique anatomique cible lorsque la caractéristique électrique mesurée par les capteurs électriques proximaux et distaux (321 ; 322) a la même amplitude environ.

6. Appareil selon la revendication 5, dans lequel l'orifice d'injection (330) est aligné avec la caractéristique anatomique cible lorsque la caractéristique électrique mesurée par les capteurs électriques proximaux et distaux (321 ; 322) indique que les capteurs électriques proximaux et distaux (321 ; 322) sont en contact physique avec la caractéristique anatomique cible.

7. Appareil selon la revendication 6, dans lequel l'orifice d'injection (330) est aligné avec la caractéristique anatomique cible lorsque l'amplitude de la caractéristique électrique mesurée par les capteurs proximaux et distaux (321 ; 322) est supérieure à une amplitude de référence respective.

8. Appareil selon la revendication 5, dans lequel :
le capteur électrique distal est positionné plus près de l'extrémité distale effilée que le capteur électrique proximal ; et
l'orifice d'injection (330) est aligné avec la caractéristique anatomique cible lorsque la caractéristique électrique mesurée par le capteur électrique distal indique que le capteur électrique distal a dépassé la caractéristique anatomique cible et que la caractéristique électrique mesurée par le capteur électrique proximal indique que le capteur électrique proximal n'est pas en contact physique avec la caractéristique anatomique cible.

9. Appareil selon la revendication 1, dans lequel la caractéristique électrique comprend une résistance ou une impédance.

10. Appareil selon la revendication 1, dans lequel l'extrémité proximale est évasée.
